# EUROPEAN PATENT APPLICATION

(11) **EP 4 389 728 A1**
(43) Date of publication of application: **26.06.2024**
(21) Application number: 21954167.9
(22) Date of filing: 18.08.2021
(51) Int. Cl.: C07C 7/148, C07C 9/22, C11C 3/10

(54) **METHOD FOR PRODUCING PLANT-DERIVED SOLID PARAFFIN AND PLANT-DERIVED SOLID PARAFFIN**

(71) Applicant: Phytochem Products Inc., Sendai-shi, Miyagi 980-0845 (JP); Tohoku University, Sendai-shi, Miyagi 980-8577 (JP)
(72) Inventor: HIROMORI Kousuke, Sendai-shi, Miyagi 980-8577 (JP); KITAKAWA Naomi, Sendai-shi, Miyagi 980-8577 (JP); KATO Makiko, Sendai-shi, Miyagi 980-0845 (JP); OYANAGI Tomokatsu, Sendai-shi, Miyagi 980-0845 (JP)
(74) Representative: Plougmann Vingtoft a/s
(86) International application number: PCT/JP2021/030104
(87) International publication number: WO 2023/021593

(57) **Abstract**

[Problem] Provided are a method for producing a plant-derived solid paraffin and a plant-derived solid paraffin, which make it possible to reduce the production cost.

[Solution] An alcohol is added to a vegetable oil to obtain a fatty acid ester, and then a solid paraffin is obtained from the fatty acid ester. The fatty acid ester is preferably obtained in the presence of an acid catalyst or a base catalyst. After obtaining the fatty acid ester, the fatty acid ester is preferably cooled to precipitate a solid, followed by separation of the solid to obtain a solid paraffin. The solid paraffin obtained comprises a saturated hydrocarbon(s) having an odd number(s) of carbon atoms of C₂₁ to C₂₉ in a proportion of not less than 60%.

## Description

### Technical Field

The present invention relates to a method for producing a plant-derived solid paraffin, and a plant-derived solid paraffin.

### Background Art

Solid paraffins are mainly composed of saturated hydrocarbons represented by CₙH₂ₙ₊₂, and have been widely used for daily life products, such as candles; crayon materials; electrical insulation materials; raw materials included in cosmetics and pharmaceuticals; moisture and water proofing agents; and surface coatings for wrapping paper, etc. The solid paraffins contained in these products are produced by separation and purification of distillate oils obtained by distillation of petroleum under reduced pressure.

Solid paraffins are also found in deodorizer distillates obtained as by-products in the production process of soybean oils, and in rice bran. These solid paraffins are thought to be originally present in plant tissues, and to play roles in protecting the tissues from desiccation and insect damage.

Conventional methods for obtaining such paraffins present in plants include those using saponification, concentration by distillation, solvent extraction, and separation by column chromatography. For example, there is a case where a deodorizer distillate of a soybean oil was subjected to distillation, saponification, and column chromatography to separate and identify hydrocarbons, tocopherols, and sterols, to detect linear saturated hydrocarbons (*n*-paraffins) (see, for example, Non-Patent Literature 1). The *n*-paraffins detected by this method were found to account for 4.0 to 4.2% of the total hydrocarbons, and to have a melting point of 67.5°C. They were found to contain *n*-C₃₁H₆₄ (65%), *n*-C₂₉H₆₀ (27%), and *n*-C₃₀H₆₂ (8%) as major components, and also *n*-C₂₇H₅₆, *n*-C₂₈H₅₈, and *n*-C₃₂H₆₆ in minor amounts.

There is also a case where a deodorizer distillate of a soybean oil was subjected to saponification, distillation, and column chromatography to separate and identify paraffins having melting points of 68.0 to 68.5°C and a molecular weight of 435 (see, for example, Non-Patent Literature 2). The paraffins identified by this method were found to contain *n*-C₃₂H₆₆ (about 60%) and *n*-C₃₀H₆₂ (about 30%) as major components, and also *n*-C₂₈H₅₈, *n*-C₂₉H₆₀, and *n*-C₃₁H₆₄ in minor amounts.

There is also a case where rice bran was subjected to solvent extraction and column chromatography, to separate and identify steryl esters, long-chain alkyl esters, short-chain alkyl esters, and hydrocarbons as wax lipids, and to detect linear alkanes (paraffins), linear alkenes, and branched alkenes (squalene) (see, for example, Non-Patent Literature 3). The major alkanes detected by this method were found to have numbers of carbon atoms of C₂₉ and C₃₁, and the major alkenes have been found to have numbers of carbon atoms of C₂₉, C₃₁, and C₃₃.

The solid paraffins contained in the deodorizer distillates of soybean oils and in rice bran, detected in Non-Patent Literatures 1 to 3 have melting points equivalent to those of petroleum-derived solid paraffins. However, while petroleum-derived solid paraffins have a wide range of numbers of carbon atoms including even and odd numbers, these plant-derived solid paraffins have mainly odd numbers of carbon atoms, and hardly any even numbers of carbon atoms.

Examples of a method for obtaining a solid or liquid paraffin from a vegetable oil, other than saponification, distillation, and column chromatography, include a method in which hydrodeoxygenation treatment is carried out in the presence of a catalyst. For example, there is a method in which a mixed fatty acid derived from a vegetable oil and an animal oil/fat was subjected to hydrodeoxygenation treatment in the presence of a sulfided NiMo/Al₂O₃-P catalyst, which is a sulfided catalyst, to produce an *n*-paraffin mixture of *n*-C₁₇H₃₆, *n*-C₁₈H₃₈, *n*-C₁₅H₃₂, and *n*-C₁₆H₃₄ (see, for example, Non-Patent Literature 4). Further, a method in which a C₁₂-C₁₄ vegetable oil such as a palm kernel oil, an ouricuri oil, or a babassu oil is subjected to hydrogenation treatment using a sulfided NiMo or sulfided CoMo catalyst, which is a sulfided catalyst, to produce C₁₀-C₁₃ *n*-paraffins has been developed (see, for example, Patent Literature 1).

Known examples of plant-derived waxes include rice bran wax, carnauba wax, candelilla wax, and soy wax. These are substances containing ester compounds (R-COO-R', where R and R' are hydrocarbon groups) as major components, and are physically and chemically different from the solid paraffins in the present description.

Candelilla wax is extracted from the stem of the candelilla plant, not from a vegetable oil. In addition, candelilla wax contains 24 to 30% ester compounds and 40 to 50% hydrocarbons. These hydrocarbons contain 76 to 80% C₃₁ (see, for example, Non-Patent Literature 5), and have been used as a candelilla wax hydrocarbons. Candelilla is a plant protected by the Washington Convention.

### Citation List

### Patent Literature

Patent Literature 1: US 8067657 B

### Non-Patent Literatures

Non-Patent Literature 1: C.D. Evans et al., "Soybean unsaponifiables: Hydrocarbons from deodorizer condensates", Journal of the American Oil Chemists' Society, 1964, 41, p. 406
Non-Patent Literature 2: Yamada, "Hydrocarbons in the Deodorizer Scum of Soybean Oil", Journal of the Japan Oil Chemists' Society, 1964, 13, p. 321
Non-Patent Literature 3: Ito et al., "Wax Lipid in Rice Bran", Nippon Nogeikagaku Kaishi, 1981, 55, p. 247
Non-Patent Literature 4: J. Hansok et al., "Bio-Paraffin Mixture Production from Waste Fatty Acid Mixture", Chemical Engineering Transactions, 2018, 65, p. 373
Non-Patent Literature 5: G. A. Scora et al., "Epicuticular hydrocarbons of candelilla (Euphorbia antisiphylitica) from three different geographical areas", Industrial Crops and Products, 1995, 4, p. 179

### Summary of the Invention

### Problems to be Solved by the Invention

The methods for producing a plant-derived solid paraffin described in Non-Patent Literatures 1 to 3 utilize the saponification, concentration by distillation, solvent extraction, and separation by column chromatography. As a result, the processes are complex and production costs are high, which has been problematic. In addition, since the methods of producing a plant-derived solid paraffin described in Non-Patent Literature 4 and Patent Literature 1 utilize hydrodeoxygenation reaction using a catalyst, the reaction must be conducted at high temperature and pressure, resulting in high production costs, which has been problematic. Thus, due to the high production costs, the conventional methods have not been used for the industrial production of plant-derived solid paraffins.

The present invention has been made focusing on such problems, and an object of the present invention is to provide a method for producing a plant-derived solid paraffin and a plant-derived solid paraffin, which make it possible to reduce the production costs.

In order to achieve the above object, in the method for producing a plant-derived solid paraffin according to the present invention, an alcohol is added to a vegetable oil to obtain a fatty acid ester, and then a solid paraffin is obtained from the fatty acid ester.

In the method for producing a plant-derived solid paraffin according to the present invention, the conversion of the vegetable oil into the fatty acid ester lowers the melting point, so that solidification of the fatty acid contained in the vegetable oil at room temperature can be prevented. Therefore, the resulting fatty acid ester is a liquid form, and thus only hydrocarbons in the fatty acid ester can be easily precipitated. By separating the resulting solid from the liquid, a solid paraffin can be obtained. Thus, the method for producing a plant-derived solid paraffin according to the present invention makes the production process simpler than the conventional methods using saponification, distillation, and column chromatography. Furthermore, since the present method does not require high temperature and high pressure, the energy consumption and the production cost for the production can be greatly reduced.

According to the method for producing a plant-derived solid paraffin according to the present invention, the production cost can be reduced to enable low-cost production of a plant-derived solid paraffin. This enables industrial production of plant-derived solid paraffins, which makes it possible to use the plant-derived solid paraffins in a wide range of applications, including both basic research and industrial use. Thus, by the method for producing a plant-derived solid paraffin according to the present invention, plant-derived solid paraffins can be obtained industrially as alternatives to petroleum-derived solid paraffins. In the method for producing a plant-derived solid paraffin according to the present invention, the solid paraffin obtained may be washed with a solvent as appropriate in order to increase its purity (quality).

In the method for producing a plant-derived solid paraffin according to the present invention, the fatty acid ester is preferably obtained in the presence of an acid catalyst or a base catalyst, especially in the presence of a solid acid catalyst or a solid base catalyst. In this case, the fatty acid ester can be obtained by an esterification reaction catalyzed by the acid catalyst, or by a transesterification reaction catalyzed by the base catalyst. Furthermore, in cases where a solid catalyst is used, the catalyst can be recovered together with a solid paraffin during the process of obtaining the solid paraffin from the fatty acid ester.

In the method for producing a plant-derived solid paraffin according to the present invention, the fatty acid ester obtained is preferably cooled to precipitate a solid, followed by separation of the solid to obtain the solid paraffin. In this case, only hydrocarbons are precipitated simply by cooling the liquid fatty acid ester, so that a solid paraffin can be easily obtained.

In the method for producing a plant-derived solid paraffin according to the present invention, the cooling temperature for the fatty acid ester is preferably -10°C to 25°C. At a temperature lower than -10°C, solidification of the fatty acid ester also occurs, while at a temperature higher than 25°C, solidification of the paraffin does not occur. Therefore, the concentration of the solid paraffin contained in the precipitated solid can be increased by cooling from -10°C to 25°C. The cooling temperature is more preferably -5°C to 15°C, and even more preferably 0°C to 10°C. In these cases, the amount of the fatty acid ester that solidifies during cooling can be reduced, and in addition, the amount of the precipitated paraffin can be increased, so that the concentration of the solid paraffin can be further increased.

In the method for producing a plant-derived solid paraffin according to the present invention, the vegetable oil preferably contains at least one of a crude oil, a deodorizer distillate, and a fatty acid oil. In this case, the plant-derived solid paraffin can be efficiently produced because the crude oil, the deodorizer distillate, and the fatty acid oil contain a relatively large amount of paraffin. The vegetable oil may be one derived from any plant-related source such as a seed or pulp of palm, soybean, rapeseed, or the like; or rice bran.

In the method for producing a plant-derived solid paraffin according to the present invention, the alcohol is preferably added to the vegetable oil such that the amount of the alcohol is 0.5 to 10 molar equivalents relative to the fatty acid contained in the vegetable oil. In cases where the amount of the alcohol is less than 0.5 molar equivalent, the amount of the fatty acid ester obtained decreases, resulting in a decrease in the amount of paraffin precipitated. On the other hand, in cases where the amount of the alcohol is more than 10 molar equivalents, the amount of the solution increases, leading to difficulties in its handling, so that the production cost increases. The amount of the alcohol is more preferably 1 to 5 molar equivalents, even more preferably 3 to 5 molar equivalents. In these cases, the amount of paraffin precipitated can be increased while reducing the production cost.

In the method for producing a plant-derived solid paraffin according to the present invention, any alcohol may be used, and the alcohol is preferably a C₁-C₈ alcohol, more preferably a linear Ci-Cs alcohol in terms of the carbon chain length. In cases where the carbon chain length of the alcohol is greater than Cs, the hydrophobicity increases, resulting in a decrease in the amount of fatty acid ester obtained and thus a decrease in the amount of paraffin precipitated. Even more preferably, the alcohol is a linear C₂-C₄ alcohol. In this case, the melting point of the fatty acid ester decreases, so that the amount of the fatty acid ester solidifying on cooling can be reduced, resulting in an increased concentration of the solid paraffin.

The plant-derived solid paraffin according to the present invention comprises a saturated hydrocarbon(s) having an odd number(s) of carbon atoms of C₂₁ to C₂₉ in a proportion of not less than 60%. In particular, the plant-derived solid paraffin according to the present invention preferably comprises a saturated hydrocarbon(s) having an odd number(s) of carbon atoms of C₂₁ to C₂₉ in a proportion of not less than 80%.

The plant-derived solid paraffin according to the present invention comprises a saturated hydrocarbon(s) having an odd number(s) of carbon atoms as a main component(s), and hardly comprises saturated hydrocarbons having even numbers of carbon atoms. This is in sharp contrast to the fact that petroleum-derived solid paraffins contain a wide range of saturated hydrocarbons having both even and odd numbers of carbon atoms, showing a broad distribution of the number of carbon atoms.

The plant-derived solid paraffin according to the present invention is preferably produced by the method for producing a plant-derived solid paraffin according to the present invention. In this case, the plant-derived solid paraffin can be produced at a low cost, and has almost the same physical properties (for example, melting point) as those of the conventional petroleum-derived solid paraffins. Therefore, the plant-derived solid paraffin can be used as an alternative to a petroleum-derived solid paraffin.

According to the present invention, a method for producing a plant-derived solid paraffin and a plant-derived solid paraffin, which makes it possible to reduce the production costs; can be provided.

### Brief Description of the Drawing

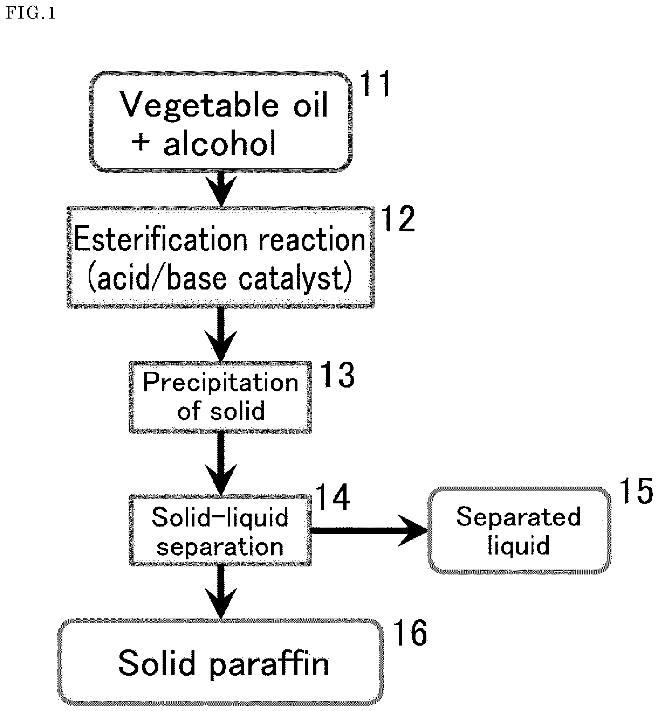

Fig. 1 is a flowchart showing a method for producing a plant-derived solid paraffin of an embodiment of the present invention.

### Detailed Description of the Invention

An embodiment of the present invention is described below by way of a drawing and Examples.

Fig. 1 shows a method for producing a plant-derived solid paraffin of an embodiment of the present invention.

As shown in Fig. 1, in the method of producing a plant-derived solid paraffin according to the embodiment of the present invention, an alcohol is added to a raw material vegetable oil (Step 11), and then an esterification reaction using an acid catalyst or a transesterification reaction using a base catalyst is carried out to obtain a fatty acid ester (Step 12).

The raw material vegetable oil may be one derived from any plant-related source such as a seed or pulp of palm, soybean, rapeseed, or the like; or rice bran. The raw material vegetable oil preferably contains at least one of a crude oil, a deodorizer distillate, and a fatty acid oil containing a relatively large amount of paraffin. The alcohol may be any alcohol, and its amount relative to the fatty acid contained in the raw material vegetable oil is preferably 0.5 to 10 molar equivalents. The alcohol is preferably a C₁-C₈ alcohol in terms of the carbon chain length. From the viewpoint of the recovery efficiency, the acid catalyst or base catalyst is preferably a solid. The method of contacting the raw material vegetable oil with the alcohol is not limited and may be, for example, a batch method (batch process) or a continuous method (flow process).

After obtaining the fatty acid ester, the fatty acid ester is cooled to precipitate a solid (Step 13), and then the precipitated solid is separated from the liquid (Step 14). The cooling temperature is preferably -10°C to 25°C. The solid-liquid separation may be carried out by any method, and examples of the method include filtration, centrifugation, or sedimentation using an apparatus such as a press filter, a filter press, a vacuum filter, a rotary drum, a centrifuge (batch or continuous type), or a sedimentation tank.

Of the separated liquid (Step 15) and the solid obtained by the solid-liquid separation, the solid is collected to obtain a solid paraffin (Step 16). Thus, a plant-derived solid paraffin of an embodiment of the present invention can be obtained. The solid paraffin obtained comprises a saturated hydrocarbon(s) having an odd number(s) of carbon atoms of C₂₁ to C₂₉ in a proportion of not less than 60%, and hardly comprises saturated hydrocarbons having even numbers of carbon atoms. This is in sharp contrast to the fact that petroleum-derived solid paraffins contain a wide range of saturated hydrocarbons having both even and odd numbers of carbon atoms, showing a broad distribution of the number of carbon atoms. The solid paraffin obtained may be washed with a solvent to increase its purity (quality).

In the method for producing a plant-derived solid paraffin according to the embodiment of the present invention, the conversion of the vegetable oil to the fatty acid ester lowers the melting point, so that solidification of the fatty acid contained in the vegetable oil at room temperature can be prevented. As a result, the resulting fatty acid ester is in a liquid form, and thus only hydrocarbons in the fatty acid ester can be precipitated simply by cooling the fatty acid ester. By separating the resulting solid from the liquid, a solid paraffin can be obtained. Thus, the method for producing a plant-derived solid paraffin according to the embodiment of the present invention can make the production process simpler than the conventional methods using saponification, distillation, and column chromatography. Furthermore, since the present method does not require high temperature and high pressure, the energy consumption and the production cost for the production can be greatly reduced.

According to the method for producing a plant-derived solid paraffin according to the embodiment of the present invention, the production cost can be reduced to enable low-cost production of a plant-derived solid paraffin. This enables the industrial production of plant-derived solid paraffins and thus the use of the plant-derived solid paraffins for a wide range of applications, including both basic research and industrial use. Thus, by the method for producing a plant-derived solid paraffin according to the embodiment of the present invention, plant-derived solid paraffins can be obtained industrially as alternatives to petroleum-derived solid paraffins.

### Example 1

A solid paraffin was produced by the method for producing a plant-derived solid paraffin according to the embodiment of the present invention shown in Fig. 1. As the raw material vegetable oil, a rice bran deodorizer distillate (Sample 1) and a rapeseed deodorizer distillate (Sample 2) were used to produce the solid paraffin. Ethanol was used as the alcohol.

A commercially available porous resin PK208LH was used as an acid catalyst as shown in Table 1. PK208LH is strongly acidic, has a copolymer of styrene and divinylbenzene as a backbone, and contains a sulfonate group as a functional group. PK208LH is in the form of a gel composed of particles whose inner portions are homogeneous, and has a porous structure in which physical holes (pores) are formed. Before using the acid catalyst PK208LH, it was swollen with the alcohol (ethanol).

**[Table 1]**

| Resin | PK208LH |
|---|---|
| Type | Porous |
| Functionality | Sulfonic acid |
| Property | Strongly acidic |
| Degree of cross-linkage [%] | 4 |
| Ion-exchange capacity [mol/m³-resin] | 1200 |
| Particle size [mm] | 0.4-0.6 |

In the production of the solid paraffin, first, 0.5 kg of the raw material vegetable oil (each deodorizer distillate) was mixed with 0.3 kg of the alcohol (ethanol) (in an amount of three equivalents relative to the fatty acid groups in terms of the molar ratio of the mixture). To the resulting mixture, 0.4 kg of the swollen catalyst was added, and a batch reaction was carried out for 24 hours while maintaining the mixture at 60°C in order to cause fatty acid esterification reaction. Then, to separate the catalyst from the reaction liquid, filtration was performed through No. 3 filter paper with a particle retention capacity of 5 µm, and then the catalyst was washed using 0.5 kg of ethanol as a washing liquid to remove oil adhering to the catalyst surface.

The reaction liquid and the washing liquid separated by filtration were then cooled at 6°C overnight. The solid precipitated by the cooling was filtered through No. 3 filter paper with a particle retention capacity of 5 µm, and then the filtered solid was washed twice using 0.35 kg of ethanol as a washing liquid, followed by natural drying of the solid. Thus, a solid paraffin was obtained from each vegetable oil.

Each solid paraffin sample obtained was subjected to evaluation of melting point, purity, and composition. First, about 1 mL of each sample was placed in a 1.5-mL polypropylene microtube and heated sufficiently in a thermostat ("ND-M01", manufactured by Nissin Rika Co., Ltd.) to determine the melting point visually. Furthermore, each sample was dissolved in hexane, and then analyzed by gas chromatography using a hydrogen flame ionization detector (FID; "GC-4000 Plus", manufactured by GL Sciences Inc.) and a gas chromatograph mass spectrometer (GC-MS; "Agilent 5975C Series GC/MSD", manufactured by Agilent Technologies, Inc.), to determine the purity and the composition of the paraffin. Gas chromatography was performed using "DB-5ht" (film thickness: 0.1 µm, inner diameter: 0.32 mm, length: 15 m), manufactured by Agilent Technologies, Inc., as a column. Table 2 shows the analytical conditions for the gas chromatography.

**[Table 2]**

| | |
|---|---|
| carrier gas | He |
| split ratio | 1:10 |
| injection volume | 2.0 × 10⁻³ cm³ |
| injection temperature | 370 °C |
| detection temperature | 370 °C |
| column temperature | 80 °C→13 °C/min→353°C→12 °C/min→365 °C, 9 min |

For comparison, a petroleum-derived solid paraffin (mp. 60 to 62°C; "160-13325", FUJIFELM Wako Pure Chemical Corporation) and a candelilla wax hydrocarbon ("MD-21", Yokozeki Oil & Fat Industries Co., Ltd.) were also subjected to the same procedure to determine melting point, purity, and composition. Table 3 shows a summary of color, recovered amount, melting point, purity, total proportion of saturated hydrocarbons having odd numbers of carbon atoms to the total C₂₁-C₃₇ saturated hydrocarbons for each solid-paraffin sample. The proportion of saturated hydrocarbons having odd numbers of carbon atoms of C₂₉ or less to the total C₂₁-C₃₇ saturated hydrocarbons is also listed in Table 3.

**[Table 3]**

| | Sample 1 | Sample 2 | Comparison Sample 1: | Comparison Sample 2: |
|---|---|---|---|---|
| | | | Petroleum-derived paraffin | Candelilla wax hydrocarbon |
| Vegetable oil | Rice bran deodorizer | Rapeseed deodorizer | - | - |
| | distillate | distillate | | |
| Color | White to pale yellow with pearly luster | Milky white | White | Pale yellow |
| Recovered amount (g) | 7.2 | 18 | - | - |
| Melting point (°C) | 61 | 63 | 62 | 65 |
| Purity (%)*¹ | 89 | 61 | > 97 | 91 |
| Total proportion of saturated hydrocarbons having odd numbers of carbon atoms (%)*² | 90 | 98 | 49 | 98 |
| Proportion of saturated hydrocarbons having odd numbers of carbon atoms of C₂₉ or less (%)*3 | 85 | 93 | 37 | 3.5 |

| | | | | |
|---|---|---|---|---|
| *¹ (Total peak area of identified saturated hydrocarbons / total peak area) × 100 *² (Total peak area of C₂₁-C₃₇ saturated hydrocarbons having odd numbers of carbon atoms / total peak area of all C₂₁-C₃₇ saturated hydrocarbons) × 100 *³ (Total peak area of C₂₁-C₂₉ saturated hydrocarbons having odd numbers of carbon atoms / total peak area of all C₂₁-C₃₇ saturated hydrocarbons) × 100 | | | | |

As shown in Table 3, the solid paraffins obtained from the vegetable oils (Sample 1 and Sample 2) have almost the same melting point as that of the petroleum-derived solid paraffin (Comparison Sample 1). However, in the solid paraffins obtained from the vegetable oils, the proportion of C₂₁-C₃₇ saturated hydrocarbons having odd numbers of carbon atoms was found to be not less than 90%, and the proportion of C₂₁-C₂₉ saturated hydrocarbons having odd numbers of carbon atoms was found to be not less than 85%, indicating that these solid paraffins hardly contain saturated hydrocarbons having even numbers of carbon atoms. As for the candelilla wax hydrocarbon (Comparison Sample 2), the proportion of saturated hydrocarbons having odd numbers of carbon atoms is as high as 98%. However, its main component is C₃₁, and the proportion of saturated hydrocarbons having odd numbers of carbon atoms of C₂₉ or less was 3.5%, which is very low. It was also found that the purity of the solid paraffin obtained varies depending on the type of the vegetable oil, and that a solid paraffin can be obtained at a purity of 89% by using a rice bran deodorizer distillate as a raw material.

### [Comparative Example]

As a Comparative Example, the production of a solid paraffin without esterification was attempted. Rice bran deodorizer distillate was used as the raw material vegetable oil. Ethanol was used as the alcohol. 0.5 kg of the raw material rice bran deodorizer distillate was mixed with 0.3 kg of ethanol, and the resulting mixture was cooled at 6°C overnight. About 0.4 kg of solid was obtained.

The solid obtained was found to be a product of the dilution of the original rice bran deodorizer distillate in the ethanol, and not a product obtained by solidification of hydrocarbons. The content of paraffin in the solid obtained was below the detection limit of 0.1%. This result indicates that recovery of solid hydrocarbons was not possible in this Comparative Example due to solidification of fatty acid components.

### Industrial Applicability

The plant-derived solid paraffin produced by the method for producing a plant-derived solid paraffin according to the present invention, and the plant-derived solid paraffin according to the present invention, are expected to be highly demanded for the purpose of application to cosmetics and food, which may be brought into contact with the skin or hair, or may be taken into the body. Specific examples of such applications may include cosmetics (creams, lipsticks, hairdressing products, eye shadows, blushers, and facial masks), food (natural coating materials), stationery (crayons, pastel crayons, pencils, and paraffin paper), candle materials, coating materials, and water-proofing materials.

## Claims

1. A method for producing a plant-derived solid paraffin, the method comprising: adding an alcohol to a vegetable oil to obtain a fatty acid ester; and then obtaining a solid paraffin from the fatty acid ester.

2. The method for producing a plant-derived solid paraffin according to claim 1, wherein the fatty acid ester is obtained in the presence of an acid catalyst or a base catalyst.

3. The method for producing a plant-derived solid paraffin according to claim 1, wherein the fatty acid ester is obtained in the presence of a solid acid catalyst or a solid base catalyst.

4. The method for producing a plant-derived solid paraffin according to any one of claims 1 to 3, wherein after obtaining the fatty acid ester, the fatty acid ester is cooled to precipitate a solid, followed by separation of the solid to obtain the solid paraffin.

5. The method for producing a plant-derived solid paraffin according to any one of claims 1 to 4, wherein the vegetable oil comprises at least one of a crude oil, a deodorizer distillate, and a fatty acid oil.

6. The method for producing a plant-derived solid paraffin according to any one of claims 1 to 5, wherein the alcohol is added to the vegetable oil such that the amount of the alcohol is 0.5 to 10 molar equivalents relative to the fatty acid contained in the vegetable oil.

7. A plant-derived solid paraffin comprising a saturated hydrocarbon(s) having an odd number(s) of carbon atoms of C₂₁ to C₂₉ in a proportion of not less than 60%.

8. A plant-derived solid paraffin comprising a saturated hydrocarbon(s) having an odd number(s) of carbon atoms of C₂₁ to C₂₉ in a proportion of not less than 80%.

9. The plant-derived solid paraffin according to claim 7 or 8, produced by the method for producing a plant-derived solid paraffin according to any one of claims 1 to 6.
